# FASCICULE DE BREVET EUROPEEN

(11) **EP 0 969 883 B1**
(45) Date de publication et mention de la délivrance du brevet: **12.11.2003**
(21) Numéro de dépôt: 98932196.3
(22) Date de dépôt: 12.06.1998
(51) Int. Cl.: A61L 27/18, A61L 27/52, A61L 27/58

(54) **IMPLANT INJECTABLE PAR VOIE SOUS-CUTANEE OU INTRADERMIQUE**
SUBKUTAN ODER INTRADERMAL INJIZIERBARES IMPLANTAT IN DER PLASTISCHEN ODER WIEDERHERSTELLENDEN CHIRURGIE
IMPLANT FOR SUBCUTANEOUS OR INTRADERMAL INJECTION

(30) Priorité: 13.06.1997 FR 9707334
(43) Date de publication de la demande: 12.01.2000
(73) Titulaire: Aventis Pharmaceuticals Holdings Inc., Greenville Delaware 19801 (US)
(72) Inventeur: ASIUS, Jérôme, F-34130 Maugio (FR); FESSI, Hatem, F-69003 Lyon (FR); GOUCHET, Franck, F-45450 Donnery (FR); LAGLENNE, Bénédicte, F-34130 Maugio (FR); LAUGIER-LAGLENNE, Elisabeth, F-75008 Paris (FR)
(74) Mandataire: Vercaemer, Laurence
(86) Numéro de dépôt international: FR9801241
(87) Numéro de publication internationale: WO98056431

(56) Documents cités:
- EP-A- 0 251 695
- EP-A- 0 648 480
- EP-A- 0 711 548
- WO-A-93/13755
- WO-A-96/33751
- US-A- 5 258 028
- US-A- 5 451 406

## Description

La présente invention concerne un implant injectable par voie sous-cutanée ou intradermique, destiné à être utilisé chez l'homme en chirurgie réparatrice ou plastique et en dermatologie esthétique, pour le comblement de rides, ridules, dépressions cutanées, cicatrices d'acné et autres cicatrices, ainsi qu'en dentisterie pour le comblement des gencives.

Cet implant est constitué de microsphères ou de microparticules bio résorbables en suspension dans un gel, les microsphères ou microparticules étant constituées d'au moins un polymère choisi parmi les polymères d'acide lactique, les polymères d'acide glycolique et les polymères d'acide lactique coglycolique.

Jusqu'à ce jour, un certain nombre de produits ont été utilisés à cet effet. Chaque produit présente des avantages et des inconvénients.

Le gel de silicone (ou huile de silicone) est facile à utiliser. Cependant, on a constaté, après injection, la migration de gouttelettes de silicone dans les tissus situés au-dessous du point d'injection, par simple gravité. La silicone est fréquemment la cause d'inflammation chronique, de formation de granulomes, et même de réactions allergiques tardives. La silicone n'est pas biodégradable, et on la retrouve souvent dans le foie.

La pâte de Téflon est une suspension de particules de polytétrafluoréthylène (diamètre 10 à 100 µm) dans de la glycérine. Ce produit, dans de nombreux cas, a provoqué des infections séreuses sévères et chroniques, et a dû être retiré au bout de quelques mois des tissus dermiques et sous-dermiques pour la plupart des patients. Il a été également prouvé que des petites particules de polytétrafluoréthylène ont été retrouvées dans le foie.

Les suspensions de collagène ont été très largement utilisées dans les dix dernières années. Les résultats ont pourtant été assez décevants dans la mesure où le collagène est résorbé en 1 à 3 mois. On note également des réactions allergiques chez environ 2 % des patients. Il est enfin à noter que le collagène est d'origine bovine.

Les prélèvements biologiques du patient lui-même : l'idée était certes intéressante, mais l'expérience clinique a montré l'échec de la réimplantation des cellules graisseuses, qui sont absorbées et disparaissent en quelques semaines.

Un autre système consistait à ajouter du plasma du patient dans une gélatine de collagène d'origines bovine et porcine. Les résultats sont encore plus décevants, et le produit est d'origine animale.

Les gels de hyaluronate présentaient une bonne alternative de par leur bio-compatibilité et leur absence de toxicité. Ils sont par ailleurs largement utilisés en chirurgie oculaire. Cependant leur bio-résorbabilité rapide (maximum 2 mois) les rend inefficaces pour une utilisation en chirurgie plastique.

Les bioplastiques sont des particules de silicone polymérisées (diamètre 70 à 140 µm) dispersées dans la polyvinylpyrrolidone. Le produit a dû être retiré compte tenu de l'inflammation chronique et des réactions de rejet qu'il provoquait.

Les microsphères de polyméthylméthacrylate (PMMA) de diamètre 20 à 40 µm sont en suspension soit dans une solution de gélatine, soit dans une solution de collagène. Le PMMA n'est pas biodégradable, mais on manque de recul pour savoir ce que cet implant donne après 5 ou 6 ans. Par ailleurs le vecteur reste une solution de collagène d'origine bovine, avec les problèmes d'allergie qu'on lui connaît.

Le document WO 96 33751 décrit une composition biphasique injectable comprenant des fragments insolubles d'un hydrogel de l'acide hyaluronique ou d'un des ses sels fortement réticulé.

Le document WO 93 13755 décrit une composition injectable à base de collagène, comprenant des microcapsules d'atélocollagène ou d'un mélange d'atélocollagène et d'un polysaccharide. Il est précisé que le collagène hétérologue, c'est-à-dire d'origine bovine, est le matériau le plus performant en termes de biocompatibilité et de stabilité in vivo.

Le document EP 711 548 décrit des microdispersions bioabsorbables comprenant, d'une part, un fluide support qui est un polymère liquide et, d'autre part, un matériau particulaire spécifique choisi parmi divers homopolymères solides formés d'unités ε-caprolactone, p-dioxanone ou triméthylène carbonate, et parmi divers co-polymères solides formés d'unités de la ε-caprolactone et d'autres unités, ou d'unités de triméthylène carbonate et d'autres unités.

Le but de l'invention est de remédier aux inconvénients des produits connus.

L'invention fait usage de microsphéres ou de microparticules constituées d'un polymère neutre, sélectionné parmi les polymères d'acide lactique, d'acide glycolique et d'acide lactique coglycolique, choisi pour son innocuité et déjà utilisé largement par l'industrie pharmaceutique que ce soit par voie orale ou par voie parentérale.

L'implant selon l'invention allie la commodité d'emploi sans manipulations préalables, la seringuabilité du produit, 1a résorbabilité en un temps contrôlé du polymère comme du gel vecteur, l'absence d'allergénicité du produit, qui rend tout test préalable inutile.

Les microsphères ou microparticules doivent avoir une bio-résorbabilité contrôlée assurant un temps de résorbabilité compris entre 1 et 3 ans. Cela signifie que le polymère se dégradera après injection in situ en composés de bas poids moléculaire qui seront éliminés de l'organisme par les processus naturels. En aucun cas un implant non résorbable ne paraît souhaitable. Il s'agît toujours d'un corps étranger placé dans un tissu vivant.

Les microsphères ou microparticules sont mises en suspension dans un gel. Elles doivent avoir un diamètre supérieur à 5 µm, et de préférence supérieur à 20 µm, afin de ne pas être absorbées par les macrophages. Elles devront avoir un diamètre inférieur à 150 µm, et de préférence inférieur à 40 µm, de façon d'une part à pouvoir être injectées par une aiguille fine et d'autre part à ne pas créer d'amas granuleux sous le doigt.

Une famille de polymères répond essentiellement à la définition précédente : les polylactides (acides polylactiques ou PLA), les polyglycolides (acides polyglycoliques ou PGA) et leurs copolymères (acides polylactiques-co-glycoliques ou PLAGA).

Compte tenu des nombreuses études déjà réalisées et de la bonne connaissance des produits, en particulier en matière de fabrication de microsphères et de résorbabilité, il paraît avantageux d'utiliser un mélange d'acide polylactique (PLA) et d'acide polylactique-co-glycolique (PLAGA). Les proportions de chacun de ces deux acides permettent de déterminer la rémanence du produit.

De nombreux essais ont également conduit à privilégier un polymère constitué par un acide poly-L-lactique (cristallin), un acide poly-D-lactique (amorphe), ou un mélange de ces deux acides. Sa masse moléculaire calculée par viscosimétrie est avantageusement comprise entre 70000 et 175000 Dalton, et de préférence entre 120000 et 170000 Dalton, une viscosité intrinsèque comprise entre 3 et 4 dl/g, et de préférence entre 3,35 et 3, 65 dl/g, une rotation spécifique comprise entre -150 et -160°, un point de fusion compris entre 178,0 et 190,1°C, une chaleur de fusion comprise entre 85,0 J/g et 90, 0 J/g un pourcentage volumique de solvants résiduels < 0,01 % et un pourcentage pondérac de monomère résiduel (acide lactique) < 0,1 %. Un tel produit est disponible chez PURAC BIOCHEM à Gorinchem (Pays-Bas).

Les polymères synthétiques bio-résorbables ont été étudiés depuis une quinzaine d'années sous la direction de Michel VERT, directeur de recherches au C.N.R.S. Les premières utilisations cliniques des PLA ont débuté en 1981 pour diverses indications en traumatologie faciale. Les polymères d'acide lactique ont vu leur utilisation se systématiser dans le cadre des implants chirurgicaux bio-résorbables. Les applications médicales des PLA sont aujourd'hui diversifiées et étendues (chirurgie osseuse, chirurgie maxillo-faciale, formulations pharmacologiques à libération contrôlée : implants, microsphères, nanosphères, vaccins).

La dégradation des polymères d'acide lactique et/ou d'acide glycolique en milieu biologique se fait exclusivement par un mécanisme chimique d'hydrolyse non spécifique. Les produits de cette hydrolyse sont ensuite métabolisés puis éliminés par le corps humain. L'hydrolyse chimique du polymère est complète; elle intervient d'autant plus rapidement que son caractère amorphe est prononcé et que sa masse moléculaire est faible. Ainsi, le temps de résorbabilité peut être réglé en agissant sur la composition du mélange et/ou sur la masse moléculaire du ou des polymères. La biocompatibilité des polymères PLA et PLAGA en fait d'excellents supports pour la croissance cellulaire et la régénération tissulaire.

Les microsphères ou microparticules sont incluses dans un gel. Ce gel, utilisé comme vecteur pour maintenir les microsphères ou microparticules en suspension homogène, est résorbable en 2 mois environ, ce qui correspond au temps nécessaire à la création de fibroses autour des microsphères ou microparticules. Il est principalement constitué d'eau pour préparation injectable et d'un agent de gélification autorisé en injection : dérivés de la cellulose, et plus particulièrement carboxyméthylcellulose (CMC) à une concentration pondéral de 0,1 à 7,5 %, et de préférence de 0,1 à 5,0 %. On peut également avoir recours à l'hydroxypropylméthylcellulose (HPMC) qui est couramment utilisée en injection intra-oculaire dans le cadre des opérations de la cataracte. On peut également employer un acide hyaluronique de synthèse, utilisé pour les injections intra-oculaires et les injections sous-cutanées. Il est également possible d'utiliser des esters d'acide lactique, esters d'acide caproïque, etc.

La bonne dispersion des microsphères ou microparticules et l'homogénéité du gel seront assurées par l'utilisation d'un agent tensio-actif, choisi pour son innocuité et son emploi autorisé en sous-cutané et intra-dermique. On utilisera du polyoxyéthylène sorbitan monooléate (commercialisé sous la dénomination Tween 80) ou de l'acide pluronique.

Le produit peut être présenté en seringues stériles préremplies prêtes à l'emploi, et munies d'une aiguille, ou en flacons de suspension stérile. Il peut également être présenté dans un flacon contenant un lyophilisat accompagné d'une ampoule d'eau stérile (eau pour préparation injectable), ou dans une seringue préremplie à deux compartiments, l'un contenant le lyophilisat de microsphères ou microparticules, l'autre contenant de l'eau pour préparation injectable.

L'implant ne nécessite pas d'essai d'allergénicité. Il ne contient aucun produit d'origine animale.

Le protocole de fabrication de l'implant est décrit ci-après, dans le cas d'une suspension de microsphères prête à l'emploi.
A. Préparation de microsphères de polymère d'acide lactique. On utilise la technique classique par évaporation de solvant, ou la technique dite par précipitation contrôlée, ou toute autre technique permettant d'obtenir des microsphères de calibre voulu.
B. Préparation d'un gel de viscosité suffisante pour maintenir les microsphères en suspension. Cette viscosité sera adaptée en fonction de la granulométrie des microsphères et de la proportion de microsphères dispersées dans le gel. Cette proportion sera de 50 à 300 g/l, et de préférence de 60 à 200 g/l.
C. Répartition du gel dans les seringues ou dans les flacons, en atmosphère contrôlée (classe 10⁴).
D. Stérilisation des flacons ou des seringues, ou utilisation d'un procédé rendant le produit fini apte à être injecté par voie sous-cutanée.

On décrit ci-après le protocole de fabrication dans le cas de microparticules de PLA lyophilisées, qu'il s'agisse du polymère L, du polymère D ou d'un mélange de ceux-ci.
A. Cryobroyage du PLA sous azote gazeux filtré à 0,22 µm, à une température inférieure à -80°C, sur une grille de criblage de 100 µm.
B. Tamisage des microparticules sur tamis en acier inoxydable de 100µm.
C. Préparation du milieu de lyophilisation incluant la dissolution sous agitation de CMC (agent gélifiant), de mannitol apyrogène (agent cryoprotecteur), et de polysorbate (agent tensio-actif) dans de l'eau pour préparation injectable, filtration à 0,22 µm de la solution obtenue sous azote gazeux filtré à 0,22 µm, et stérilisation à l'autoclave pendant 20 minutes à 121,5°C.
D. Répartition des microparticules à raison de 100 mg par flacon de capacité nominale 4 ml.
E. Répartition du milieu de lyophilisation à raison de 1,05 ± 0,05 g dans les flacons contenant déjà les microparticules d'acide polylactique.
F. Dispersion des microparticules dans le milieu de lyophilisation par un système de dispersion par ultrasons, afin d'obtenir une suspension homogène.
G. Prébouchage des flacons à l'aide de bouchons à piliers (spécifiques pour la lyophilisation), congélation rapide au dessous de -70°C, stockage des flacons congelés au dessous de - 40°C, et enfin lyophilisation et bouchage automatique des flacons.
H. Capsulage et mirage des flacons, avant stérilisation par irradiation γ.

Bien entendu, il est possible de combiner les modes opératoires décrits ci-dessus, par exemple pour obtenir une suspension de microparticules prête à l'emploi, ou un lyophilisat de microsphères, les microparticules ou les microsphères étant constituées de l'un quelconque des polymères susmentionnés et de leurs mélanges.

### EXEMPLE 1

2 g de PLA sont dissous dans 20 ml d'un solvant organique (acétate d'éthyle). Cette solution est dispersée dans 100 ml d'eau contenant 5 g de polyoxyéthylène sorbitan monooléate. Une agitation modérée au vortex est maintenue jusqu'à évaporation du solvant et formation de microsphères de diamètre moyen 40 µm. Les microsphères formées sont récupérées par sédimentation, filtration et séchage. Elles sont alors incluses dans un gel constitué d'eau et de CMC (0,5 % en masse). Après agitation modérée, on procède à la répartition.

### EXEMPLE 2

2 g de PLA sont dissous dans 20 ml d'un solvant organique (chlorure de méthylène). Cette solution est dispersée dans 100 ml d'eau contenant 5 g de polyoxyéthylène sorbitan monooléate. Une agitation modérée au vortex est maintenue jusqu'à évaporation du solvant et formation de microsphères de diamètre moyen 80 µm. Les microsphères formées sont récupérées par sédimentation, filtration et séchage. Elles sont alors incluses dans un gel constitué d'eau et de CMC (0,5 % en masse). Après agitation modérée, on procède à la répartition.

### EXEMPLE 3

2 g de PLA sont dissous dans 20 ml d'un solvant organique (chloroforme). Cette solution est dispersée dans 100 ml d'eau contenant 5 g de polyoxyéthylène sorbitan monooléate. Une agitation modérée au vortex est maintenue jusqu'à évaporation du solvant et formation de microsphères de diamètre moyen 50 µm. Les microsphères formées sont récupérées par sédimentation, filtration et séchage. Elles sont alors incluses dans un gel constitué d'eau et de HPMC (1 % en masse). Après agitation modérée, on procède à la répartition.

### EXEMPLE 4

On procède au cryobroyage de 600 g d'acide polylactique, à une granulométrie finale comprise entre 20 et 100 µm, avec une médiane à 40 µm. Ces microparticules sont réparties à raison de 100 mg par flacon.

On fabrique 6,5 kg de milieu de lyophilisation en dissolvant 97,5 g de CMC sodique, 276,25 g de mannitol apyrogène, et 6,5 g de polysorbate 80 dans qs 6,5 litres d'eau pour préparation injectable. Ce milieu est réparti à raison de 1 g par flacon.

Des essais sur l'animal (souris hairless et lapins néo-zélandais) ont été réalisés avec les produits des exemples 1 à 4. Les résultats sont identiques, et on observe durant les premiers deux mois, et dès le huitième jour après l'injection, l'apparition de cellules géantes entourant en réseau les cristaux d'acide polylactique puis leur transformation par création d'une fibrose qui reconstitue le tissu sous-cutané.

## Revendications

1. Implant injectable pour l'administration humaine constitué de microsphères ou microparticules bio-résorbables en suspension dans un gel, les microsphères ou microparticules étant constituées d'au moins un polymère choisi parmi les polymères d'acide lactique, les polymères d'acide glycolique et les polymères d'acide lactique coglycolique.

2. Implant selon la revendication 1, **caractérisé en ce que** la proportion de microsphères ou microparticules dans le gel est de 50 à 300 g/l, et de préférence 60 à 200 g/l.

3. Implant selon l'une ou l'autre des revendications 1 et 2, **caractérisé en ce que** les microsphères ou microparticules ont un diamètre moyen de 5 à 150 µm, et de préférence de 20 à 40 µm.

4. Implant selon l'une quelconque des revendications 1 à 3, **caractérisé en ce que** les microsphères ou microparticules sont biorésorbables en une période de 1 an à 3 ans.

5. Implant selon l'une quelconque des revendications 1 à 4, **caractérisé en ce que** ledit polymère est un acide polylactique choisi parmi l'acide poly-L-lactique, l'acide poly-D-lactique et les mélanges de ceux-ci.

6. Implant selon la revendication 5, **caractérisé en ce que** l'acide polylactique a une masse moléculaire comprise entre 70000 et 175000 Dalton, et de préférence entre 120000 et 170000 Dalton, une viscosité intrinsèque comprise entre 3 et 4 dl/g, et de préférence entre 3,35 et 3,65 dl/g un pourcentage pondéral de monomère résiduel inférieur à 0,1 %, et un pourcentage volumique de solvants résiduels inférieur à 0,01%.

7. Implant selon l'une quelconque des revendications 1 à 6, **caractérisé en ce que** le gel inclut principalement comme agent de gélification la carboxyméthylcellulose (CMC) ou l'hydroxypropylméthylcellulose à une concentration pondérale de 0,1 à 7,5%, et de préférence de 0,1 à 5,0%.

8. Lyophilisat obtenu par lyophilisation d'un produit selon l'une quelconque des revendications 1 à 7, et propre à reconstituer un implant injectable par adjonction d'eau pour préparation injectable.

9. Seringue stérile préremplie munie d'une aiguille, contenant un implant injectable selon l'une quelconque des revendications 1 à 7.

10. Flacon de suspension stérile, contenant un implant injectable selon l'une quelconque des revendications 1 à 7.

11. Flacon contenant un lyophilisat selon la revendication 8 accompagné d'une ampoule d'eau pour préparation injectable.

12. Seringue préremplie comprenant deux compartiments, l'un contenant un lyophilisat selon la revendication 8, l'autre contenant de l'eau pour préparation injectable.

13. Procédé de fabrication d'un implant selon l'une quelconque des revendications 1 à 7 sous forme de suspension de microsphères ou microparticules, ledit procédé comprenant les étapes suivantes :
- préparation de microsphères ou microparticules de polymère par évaporation du solvant ou précipitation contrôlée ou toute autre technique permettant d'obtenir des microsphères ou microparticules de calibre voulu,
- préparation d'un gel de viscosité suffisante pour maintenir les microsphères ou microparticules en suspension,
- répartition du gel dans les seringues ou les flacons en atmosphère contrôlée (classe 10⁴),
- stérilisation des flacons ou des seringues ou utilisation d'un procédé rendant le produit fini apte à être injecté par voie sous-cutanée.

14. Procédé de fabrication d'un implant selon l'une quelconque des revendications 1 à 7 sous forme de microsphères ou microparticules de polymère lyophilisé, ledit procédé comprenant les étapes suivantes :
- cryobroyage du polymère sous azote gazeux filtré à 0,22 µm, à une température inférieure à -80°C, sur une grille de criblage de 100 µm,
- tamisage des microparticules ou microsphères sur tamis en acier inoxydable de 100 µm,
- préparation du milieu de lyophilisation incluant la dissolution sous agitation de carboxyméthylcellulose, de mannitol apyrogène, et de polysorbate dans de l'eau pour préparation injectable, filtration à 0,22 µm de la solution obtenue sous azote gazeux filtré à 0,22 µm, et stérilisation à l'autoclave pendant 20 minutes à 121,5°C,
- répartition des microparticules ou microsphères à raison de 100 mg par flacon de capacité nominale 4ml,
- répartition du milieu de lyophilisation à raison de 1,05 ± 0,05 g dans les flacons contenant déjà les microparticules ou microsphères de polymère,
- dispersion des microparticules ou microsphères dans le milieu de lyophilisation par un système de dispersion par ultrasons, afin d'obtenir une suspension homogène,
- prébouchage des flacons à l'aide de bouchons à piliers, congélation rapide au dessous de - 70°C, stockage des flacons congelés au dessous de - 40°C, puis lyophilisation et bouchage automatique des flacons,
- capsulage et mirage des flacons avant stérilisation par irradiation γ.

## Patentansprüche

1. Injizierbares Implantat für die humane Verabreichung, bestehend aus bioresorbierbaren Mikrokügelchen oder Mikropartikeln in Suspension in einem Gel, wobei die Mikrokügelchen oder Mikropartikel aus mindestens einem Polymer gebildet werden, ausgewählt unter den Polymeren von Milchsäure, den Polymeren von Glykolsäure und den Polymeren von Milchsäure-Co-Glykolsäure.

2. Implantat nach Anspruch 1, **dadurch gekennzeichnet, daß** das Verhältnis von Mikrokügelchen oder Mikropartikeln in dem Gel 50 bis 300 g/l, vorzugsweise 60 bis 200 g/l beträgt.

3. Implantat nach dem einen oder anderen der Ansprüche 1 und 2, **dadurch gekennzeichnet, daß** die Mikrokügelchen oder Mikropartikel einen mittleren Durchmesser von 5 bis 150 µm und vorzugsweise von 20 bis 40 µm besitzen.

4. Implantat nach irgendeinem der Ansprüche 1 bis 3, **dadurch gekennzeichnet, daß** die Mikrokügelchen oder Mikropartikel innerhalb einer Periode von 1 Jahr bis 3 Jahren bioresorbierbar sind.

5. Implantat nach irgendeinem der Ansprüche 1 bis 4, **dadurch gekennzeichnet, daß** das genannte Polymer eine Polymilchsäure ist, ausgewählt unter Poly-L-Milchsäure, Poly-D-Milchsäure und deren Mischungen.

6. Implantat nach Anspruch 5, **dadurch gekennzeichnet, daß** die Polymilchsäure eine Molekularmasse zwischen 70000 und 175000 Dalton und vorzugsweise zwischen 120000 und 170000 Dalton, eine Eigenviskosität zwischen 3 und 4 dl/g und vorzugsweise zwischen 3,35 und 3,65 dl/g, einen Gewichtsprozentsatz von restlichem Monomer von unter 0,1 % und einen Volumenprozentsatz von restlichem Lösungsmittel von unter 0,01% besitzt.

7. Implantat nach irgendeinem der Ansprüche 1 bis 6, **dadurch gekennzeichnet, daß** das Gel hauptsächlich als Gelierungsmittel Carboxymethylcellulose (CMC) oder Hydroxypropylmethylcellulose in einer gewichtsmäßigen Konzentration von 0,1 bis 7,5 % und vorzugsweise von 0,1 bis 5,0 % einschließt.

8. Lyophilisat, erhalten durch Lyophilisation von einem Produkt nach irgendeinem der Ansprüche 1 bis 7, und dazu geeignet, ein injizierbares Implantat durch Zusatz von Wasser für injizierbare Präparationen wiederherzustellen.

9. Vorgefüllte sterile Injektionsspritze, die mit einer Nadel ausgestattet ist und ein injizierbares Implantat nach irgendeinem der Ansprüche 1 bis 7 enthält.

10. Fläschchen mit steriler Suspension, das ein injizierbares Implantat nach irgendeinem der Ansprüche 1 bis 7 enthält.

11. Fläschchen, das ein Lyophilisat nach Anspruch 8 enthält, begleitet von einer Ampulle mit Wasser für injizierbare Präparatiönen.

12. Vorgefüllte Injektionsspritze mit zwei Abteilen, von denen eines ein Lyophilisat nach Anspruch 8 und das andere Wasser für injizierbare Präparationen enthält.

13. Verfahren zur Herstellung eines Implantates nach irgendeinem der Ansprüche 1 bis 7 in Form einer Suspension von Mikrokügelchen oder Mikropartikeln, wobei das genannte Verfahren die folgenden Stufen umfaßt:
- Herstellung von Mikrokügelchen oder Mikropartikeln aus Polymer durch Verdampfen des Lösungsmittels oder kontrollierte Fällung oder durch jede andere technische Methode, die es ermöglicht, Mikrokügelchen oder Mikropartikel mit gewünschtem Durchmesser zu erhalten,
- Herstellung eines Gels von ausreichender Viskosität, um die Mikrokügelchen oder Mikropartikel in Suspension zu halten,
- Aufteilung des Gels in Injektionsspritzen oder Fläschchen unter kontrollierter Atmosphäre (Klasse 10⁴) ,
- Sterilisation der Fläschchen oder Injektionsspritzen oder Anwendung eines Verfahrens, das das Endprodukt geeignet macht, auf subkutanem Wege injiziert zu werden.

14. Verfahren zur Herstellung eines Implantates nach irgendeinem der Ansprüche 1 bis 7 in Form von Mikrokügelchen oder Mikropartikeln aus lyophilisiertem Polymer, wobei das genannte Verfahren die folgenden Stufen umfaßt:
- Gefrierzerkleinerung des Polymers unter gasförmigem Stickstoff, filtriert auf 0,22 µm bei einer Temperatur von unter -80 °C auf einem Siebgitter von 100 µm,
- Sieben der Mikrokügelchen oder Mikropartikel auf einem Sieb aus rostfreiem Stahl von 100 µm,
- Herstellung des Lyophilisationsmilieus, einschließend die Auflösung unter Rühren von Carboxymethylcellulose, von pyrogenfreiem Mannitol und von Polysorbat in Wasser für injizierbare Präparationen, Filtration der erhaltenen Lösung auf 0,22 µm unter gasförmigem Stickstoff, filtriert auf 0,22 µm, und Sterilisation im Autoklaven während 20 Minuten bei 121,5 °C,
- Aufteilung der Mikrokügelchen oder Mikropartikel im Verhältnis von 100 mg pro Fläschchen von der Nominalkapazität von 4 ml,
- Aufteilung des Lyophilisationsmilieus im Verhältnis von 1,05 ± 0,05 g in Fläschchen, die bereits die Mikrokügelchen oder Mikropartikel aus Polymer enthalten,
- Dispergierung der Mikrokügelchen oder Mikropartikel in dem Lyophilisationsmilieu durch ein Dispersionssystem mittels Ultraschall, um eine homogene Suspension zu erhalten,
- Vor-Verschließen der Fläschchen mit Hilfe von Stützpfropfen, schnelles Einfrieren auf unter -70 °C, Lagerung der eingefrorenen Fläschchen bei unter -40 °C und anschließende Lyophilisation sowie automatisches Verkorken der Fläschchen,
- Verkapselung und Durchleuchten der Fläschchen vor der Sterilisation durch Gammastrahlen.

## Claims

1. Injectable implant for human administration consisting of bioresorbable microspheres or microparticles in suspension in a gel, said microspheres or microparticles consisting of at least one polymer chosen from the lactic acid polymers, the glycolic acid polymers and the lactic co-glycolic acid polymers.

2. Implant according to claim 1, **characterized in that** the proportion of microspheres or microparticles in the gel is from 50 to 300 g/l, preferably 60 to 200 g/l.

3. Implant according to anyone of claims 1 and 2, **characterized in that** the microspheres or microparticles have a mean diameter of from 5 to 150 µm, preferably from 20 to 40 µm.

4. Implant according to anyone of claims 1 to 3, **characterized in that** the microspheres or microparticles are bioresorbable within a period of 1 to 3 years.

5. Implant according to anyone of claims 1 to 4, **characterized in that** said polymer is a polylactic acid chosen from poly-L-lactic acid, poly-D-lactic acid and mixtures thereof.

6. Implant according to claim 5, **characterized in that** the polylactic acid has a molecular weight of 70 000 to 175 000 Dalton, preferably 120 000 to 170 000 Dalton, an intrinsic, viscosity of 3 to 4 dl/g, preferably 3.35 to 3.65 dl/g, a percentage by weight of residual monomer below 0.1% and a percentage by volume of residual solvents below 0.01%.

7. Implant according to anyone of claims 1 to 6, **characterized in that** the gel includes mainly, as gelling agent, carboxymethylcellulose (CMC) or hydroxypropylmethylcellulose (HPMC) at a concentration by weight of 0.1 to 7.5%, preferably from 0.1 to 5.0%.

8. Freeze-dried product obtained by freeze-drying a product according to anyone of claims 1 to 7, and capable of reconstituting an injectable implant by addition of water for injection.

9. Prefilled sterile syringe provided with a needle, containing an injectable implant according to anyone of claims 1 to 7.

10. Vial of sterile suspension, containing an injectable implant according to anyone of claims 1 to 7.

11. Vial containing a freeze-dried product according to claim 8 accompanied by an ampule of water for injection.

12. Prefilled syringe comprising two compartments, one compartment containing the freeze-dried product according to claim 8, the other compartment comprising water for injection.

13. Process for the manufacture of an implant according to anyone of claims 1 to 7 in the form of a suspension of microspheres or microparticles, said process comprising the following steps:
- preparation of microspheres or microparticles of polymer by solvent evaporation or controlled precipitation or any other technique which makes it possible to obtain microspheres or microparticles of the desired size,
- preparation of a gel of sufficient viscosity to maintain the microspheres or microparticles in suspension,
- distribution of the gel into syringes or into vials in a controlled atmosphere (class 10⁴),
- sterilization of the vials or syringes or use of a process which makes the finished product suitable for injection by the subcutaneous route.

14. Process for the manufacture of an implant according to anyone of claims 1 to 7 in the form of freeze-dried polymer microspheres or microparticles, said process comprising the following steps :
- cryogrinding of the polymer under gaseous nitrogen filtered at 0.22 µm, at a temperature of less than -80°C, on a 100 µm screening grid,
- sieving of the microparticles or microspheres on a 100 µm stainless steel grid,
- preparation of the freeze-drying medium including the dissolution, with stirring, of the carboxymethylcellulose, apyrogenic mannitol and polysorbate in water for injection, filtration at 0.22 µm of the solution obtained under gaseous nitrogen filtered at 0.22 µm, and sterilization in an autoclave for 20 minutes at 121.5°C,
- distribution of the microparticles or microspheres at a rate of 100 mg per vial of 4 ml nominal capacity,
- distribution of the freeze-drying medium, at a rate of 1.05 ±0.05 g into the vials already containing the polymer microparticles or microspheres,
- dispersion of the microparticles or microspheres in the freeze-drying medium by an ultrasound dispersion system in order to obtain a homogeneous suspension,
- prestoppering of the vials using pillar stoppers, rapid freezing below - 70°C, storage of the frozen vials below - 40°C, then freeze-drying and automatic stoppering of the vials,
- fitting of capsules and examination of the vials, before sterilization by γ irradiation.
